# EUROPEAN PATENT SPECIFICATION

(11) **EP 0 739 632 B1**
(45) Date of publication and mention of the grant of the patent: **10.01.2001**
(21) Application number: 95500067.4
(22) Date of filing: 28.04.1995
(51) Int. Cl.: A61K 35/74, C12N 1/20

(54) **Process for the preparation of a medical product**
Verfahren zum Herstellen eines Arzneimittels
Procédé pour la préparation d'un produit médicamenteux

(43) Date of publication of application: 30.10.1996
(73) Proprietor: Jorge Gonzalez, Falco, 25002 Lerida (ES)
(72) Inventor: Jorge Gonzalez, Falco, 25002 Lerida (ES)
(74) Representative: Klunker . Schmitt-Nilson . Hirsch

(56) References cited:
- DE-A- 3 716 938
- DE-A- 4 103 755
- CANCER RESEARCH, vol. 53, 1993 PHILADELPHIA, pages 3914-3918, REDDY B.S. ET AL 'Inhibitory effect of Bifidobacterium longum on colon mammary, and liver carcinogenesis induced by 2-amino-3-methylimidazo[4,5-f]quinoline, a food mutagen'
- PROCEEDINGS OF THE SOCIETY FOR EXPERIMENTAL BIOLOGY AND MEDICINE, vol. 207, no. 3, 1994 NEW YORK, pages 278-283, KULKARNI, N. ET AL 'Inhibitory effect of Bifidobacterium longum cultures on the azoxymethane-induced aberrant crypt foci formation and fecal bacterial Beta-glucuronidase'
- : "Webster's third new international dictionary", , MERRIAM-WEBSTER INC, MASSACHUSETTS U.S.A.

## Description

### TECHNICAL FIELD

The present Invention reffers to a process for the preparation of a medical product for the topical treatment of neoplasia in the feminine genitals.

The process of the invention permits to industrially obtain a product which is effective for the therapeutic treatment of neoplasia of the feminine genitals.

### FIELD OF THE INVENTION

This invention is to be used within the field of the industry for the manufacture of medicinal products.

### BACKGROUND OF THE INVENTION

As background of the invention the inventors wish to recall that at present the diagnosis of pre-malignant affections of the uterus neck are based on three specific diagnosis methods for cervical neoplasia, consisting in the cervico-vaginal citology, colcospy and biopsy. The treatments which are known up to day, comprise the thermocoagulation consisting in the cauterization of the cervix up to epithelium depth, criotherapics, consisting in the freezing and destruction of the epitelium, laser applications with the same treatment objective as above with the use of laser beams with a high penetration capacity and minimum side effects and the cervical conization consisting in the surgical resection of the affected parts of the uterus.

At present, the remarkable increase in the incidence of this pathology in teenagers and the increase of affections produced by sexual transmission and more specifically of pelvic inflammatory illness enhance the need of a treatment which is less agresive than those conventionally practiced because none of them prevent the cause which introduced the noxe and subsequently they give ground to secondary affections after the destruction of the uterus neck as lack of fertility, destruction of endocervical cripts, etc.

By the end of last century a theory was developped for obtaining "eternal youth" based on the observation that one of the longest living population groups had as a feeding base fermented milk products.

The theory was partly destroyed because the fermentation of the milk in the concerned areas was produced by a specific bacteria and, on the other side the generalization of theory meant its own self destruction.

Some investigations have been made for the treatment of vaginal affections using bacterial products such as yogourt and some investigators have proposed the vaginal recolonization using fermented milks. However, the clinical tests have not been satisfactory, probably due to the ignorance of the virical ethiologic factors and apart from the treatment of vaginosis with yogourt and some similar products hardly any progress has been obtained in this field. Some pharmaceutical laboratories have made tests and have proposed treatments comprising lactobacilus as main element, based on the characteristics of Doderlëin flora.

These investigations have dealt with the application as coadjuvants in the treatment of infections in the digestive tract but they have not been successful in the investigations at the genital level. The resistance of the yogourt bacteria to antibiotics as well as their capacity for assimilating cholesterol are well known.

Cancer Research, 53, 3914-3918, 1993 discloses the inhibitory effect of B. longum on carcinogenesis induced by a food mutagen.

The obvious solution to the above problems would consist in providing a product that acting as inhibitory of viral growth it could enhance, because of its characteristics, a physiologic vaginal ecology, restoring the loss of the same. However, no effective process or product exists at present for that application.

### SUMMARY OF THE INVENTION

The process of the present invention is aimed at the preparation of a medical product for the topical treatment of neoplasia of the feminine genitals. To this end an investigation of the vaginal floral is carried out in the first place, starting from the base of non existence of trivial flora except lactobacilus and disregarding any other bacteria, as Doderlëin flora.

In the investigations which has been carried out, giving ground to this invention, it is observed that biochemically another bacterium has been found which seems to have the same characteristics as lactobacilus, being known as bifidobacterium, which never grows in case that the vaginal medium shows the existence of virus and on the contrary, in case of existence of said bacterium, the virus are not able to colonize.

As a summary, it may be stated that the invention is based on the use of bifidobacteriae and specifically on bifidobacterium longum.

The process for the preparation of the medical product of this invention, essentially comprises the association of the following products as components with the application of the operative stages to be explained, up to the obtainment of the objective product.

Bifidobacterium longum is associated with a concentration of 1.000.000.000 to 4 milligrammes of glycogen, used as a nutrient and 2 milligrammes of lactoproteins, all of them in solid state.

To the above association, 100 millilitres of distilled water are added as well as 0,5 grammes of glucose and 0,45 grammes of sodium chloride at 37ºC temperature and pH 7.

Acetic acid is further added in a rate of 1,5 to 2,0 millilitres per 100 millilitres up to obtaining pH 4. The optimum activity is obtained between 24 and 48 hours after the preparation.

The bifidobacterium longum acts as inhibitor of viral growth enhancing physiological vaginal ecology and restoring the loss of the same. The glycogen acts as nutrient for the bifidobacterium and forces the transfer of the same to the lactic acid. As reducing substance vitamin C may be used.

Glucose acts as a nutrient and acetic acid acts as buffer increasing the efficiency of the solution and acting as well as an antiviral agent through the inhibition of the growth.

The bifidobacterium may be obtained from fermented milk adding acetic acid to the same. This blend may be used as a product for direct vulvo-vaginal flushing.

The extraction of the bifidobacterium may be carried out as well starting from fermented milk creating an aerobic-anaerobic interface, subsequently proceding to its separation. It may be obtained "de novo" by growth in strictly anaerobic agar chocolate mediums.

The product obtained will be used for advanced stage of pre-malignant affections and in neoplasies, consisting in a solution with glucosated serum with pH 7, obtaining the same from fermented milk and after producing the interface it will be reduced to pH 4 using acetic acid applying the solution directly to the uterus neck through the vagina by irrigation. In order to prevent any digestive irritation those products will be used simultaneously per os.

The treatment lasts twenty days.

The side effects may be the same as those appearing with any antiviric antibiotic treatment, that is, vaginal candidiasis which manifestation would make it advisable to temporarily suspend the treatment.

As in other antiviric treatments, the described process will provoke a change at the cervical level, changing the lynfocytes into segmentated, which will appear in substantial quantities provoking an image which corresponds to an inflammatory acute frotis. After this the regulation of the interferon will be started, inhibiting the viric and neoplasic growth. Afterwards a revision of the treatment must be made, approximately twenty days after being carried out.

It may be used as well in form of vulvo-vaginal flushings given the characteristic of said bacterium of producing vitamins, which would alleviate certain non specific pruritus which ethiology is difficult in many cases to attribute to the existence of some specific, metabolic, irritating or infectious agent.

The process for the preparation of the medical product of this invention contemplates as well for its specific ovulo-vaginal application, the addition of cellulose and the heating up to 45ºC which is not detrimental to the characteristics of the bacterium.

The treatment of neoplasia of the feminine genital aparattus using the product of the present invention may be considered to take place according to the following stages:
Recolonization of the digestive flora.
Acid and/or specific antibiotic therapy.
Antiinflammatory activity.
Support of the ovulation.
Vaginal recolonization.
Elimination of irritating factors.

The first stage implies the intake of yogourt with bifidobacteriae by the patient after fasting or during the morning and the afternoon periods with the aim to promote the passage of the bifidobacteriae to the vagina as the natural habitat of said bacterium is the digestive tractus. The yogourt with bifidobacteriae additionally contains lactobacilus which show the characteristic of inhibiting the growth of the flora and configurate a typical element of the Doderlëin bacilum. In this way a natural reserve and recolonization are insured. The acidity or pH of the yogourt with bifidobacteriae is of pH 4,5 equivalent to that of the application area of such product, the vagina. The dates for optimum activity and caducity of the product have to be taken into account as well as its conservation at low temperature up to the moment of its application.

The yogourt with bifidobacteriae has a caducity of 24 days after its preparation. 10 days before that date the number of bacteriae starts lowering. This is the reason why it shoud be used 10 days before its expiry date. It must be conserved at a low temperature as on the contrary it will become useless.

To this end, it should be purchased from selling points renewing stocks every day or maintaining the freezers open during the nights.

Concerning acid and/or specific antibiotic therapy it has to be pointed out that for tricomonae and clamidiae specific antibiotics must be furnished to the patients. Within this field of products the triad formed by clavulanic, vibracine and local metronidazol is available. All of these except the last mentioned may be substituted by the citroflosacin in case that the vaginal bacteriae are sensible to this antibiotic and the patient does not suffer from diabetics. In case that some of the germs are resistant or the patient is affected by diabetics, recourse will have to be made to the above mentioned triad. However if the existence of fungus is observed, these must be eliminated by means of clotrimoxazol.

concerning anti-inflammatory products, in the first place a mild inhibition of prostaglandins has to be carried out proposing to that end 0,5 grammes of acetil salicilic acid with the addition of vitamin C, which insures its inhibition and urinary acidification as it is well known that all chronic infection of the vaginosis or vaginitis type produces inflammation.

To ensure ovulation a natural or type G acid mucus must be created in the case that the ovulation or the secretion of mucus G is insured by means of gestagens. Endocervical pH will be maintained at acid levels. In the case that it attains a alcaline level, they will be high endocervical, given the fact that mucus G is secreted in the lower cripts. During the non ovulatory cycles in absence of the mucus, a treatment with anti-strogenic agents has to be carried out, that is, with clomifene or tamoxifene. In case of prepuberal ovaries or hipotalamic deficiences, an stimulation through the hipotalamus must be carried out with HMG-HCG.

The vaginal recolonization will be carried out by means of acetic acid and/or lactic acid flushings, that is, acidification of the perineum, inhibition of vulva virus and coagulation of the mucus. The introduction of the lactic product, specifically yogourt with bifidobacteriae in the vagina is aimed at a subsequent recolonization because acetic acid may eliminate the flora. The optimum region for the treatment is the external third where the highest quantity of glycogen has been demonstrated, existing the highest probabilities for action.

This treatment has to be carried out in the period in which no menstruation exists, as the cervical orifice is open with possibility of passage to the endometrium. This treatment has not been found to be dangerous. The acetic acid must have a concentration of 3 to 5%.

Concerning the elimination of the inflammatory factors, the D.I.U. must be obviated because of the danger of contamination. At the same time, sexual relations must be canceled due to the effects of semen and similar factors. This mesures should be taken whenever possible.

With the beginning of the cycle the three first points must be introduced insuring the ovulation to take place in the most convenient time according to the treatment and starting vaginal recolonization after the menstrual period has ended. It should be taken into account that if the day of the ovulation is previously known, no introduction of the yogourt with bifidobacteriae within the vagina will be made during that day.

Incidentally, it has to be pointed out that the ciproterone acetate could be used as gestagene, preventing in this way, apart from treating the typical symptomatology of those women, the action on the axis inducing ovulation.

In this conection it has to be mentioned that vitamins A, B, C and E, carotenes, could be used as suplements for the treatment as well as some minerals and enzymes, specifically seleniun and glutation, which have proved to be effective.

Obviously, the ideal treatment consists in the typification of the virus and the vaccine against the same. However the same as with interferon and retinoic acid they cannot be used at present in clinic therapeutics.

As a summary, taking into account the above stages for the treatment of neoplasia of the genital aparattus as well as the products to be used, the adequate elimination of said affection will be obtained.

## Claims

1. Process for the preparation of a medical product for the topical treatment of neoplasia of feminine genitals, characterized by comprising the association of 1.000.000.000 bifidobacterium longum with 4 milligrammes of glycogen as nutrient and 2 milligrammes of lactoproteins, all of them in solid state; addition of those components to 100 millilitres of distilled water, 0,5 grammes of glucose and 0,45 grammes of sodium chloride at a temperature of 37ºC and acidity pH 7 with further addition of acetic acid up to an acidity of pH 4 and further addition of cellulose with heating to a temperature of 45ºC.

2. Process for the preparation of a medical product, according to claim 1, characterized in that for the inhibition of prostaglandins, 0,5 grammes of acetyl salicylic acid and 1 gramme of vitamin C will be associated to the product.

3. Use of bifidobacterium longum for the preparation of medicaments for topical treatment of neoplasia of feminine genitals.

## Patentansprüche

1. Verfahren zur Herstellung eines medizinischen Produkts zur örtlichen Behandlung von Neoplasie der weiblichen Genitalien, **dadurch gekennzeichnet,** daß es das Vereinigen von 1.000.000.000 Bifidobacterium longum mit 4 Milligramm Glykogen als Nährstoff und 2 Milligramm Lactoproteinen, von denen alle im festen Zustand sind, die Zugabe dieser Bestandteile zu 100 Millilitern destilliertem Wasser, 0,5 g Glukose und 0,45 g Natriumchlorid bei einer Temperatur von 37°C und einer Acidität von pH 7, mit weiterer Zugabe von Essigsäure bis zu einer Acidität von pH 4 und weiterer Zugabe von Cellulose unter Erwärmen auf eine Temperatur von 45°C aufweist.

2. Verfahren zur Herstellung eines medizinischen Produkts nach Anspruch 1, **dadurch gekennzeichnet,** daß zur Hemmung von Prostaglandinen 0,5 g Acetylsalicylsäure und 1 g Vitamin C mit dem Produkt vereinigt werden.

3. Verwendung von Bifidobacterium longum zur Herstellung von Arzneimitteln zur örtlichen Behandlung von Neoplasie der weiblichen Genitalien.

## Revendications

1. Procédé pour la préparation d'un produit médicamenteux destiné au traitement topique de la néoplasie des organes génitaux féminins, caractérisé par le fait de comprendre l'association de 1 000 000 000 bifidobacterium longum avec 4 milligrammes de glycogène comme agent nutritif et 2 milligrammes de lactoprotéines, la totalité de ceux-ci à l'état solide ; l'addition de ces composants à 100 millilitres d'eau distillée, 0,5 gramme de glucose et 0,45 gramme de chlorure de sodium à une température de 37°C et à une acidité de pH 7 avec l'addition supplémentaire d'acide acétique jusqu'à une acidité de pH 4 et l'addition supplémentaire de cellulose avec chauffage jusqu'à une température de 45°C.

2. Procédé pour la préparation d'un produit médicamenteux selon la revendication 1, caractérisé en ce que, pour l'inhibition des prostaglandines, on associe au produit 0,5 gramme d'acide acétylsalicylique et 1 gramme de vitamine C.

3. Utilisation de bifidobacterium longum pour la préparation de médicaments destinés au traitement topique de la néoplasie des organes génitaux féminins.
